# EUROPEAN PATENT SPECIFICATION

(11) **EP 2 988 816 B1**
(45) Date of publication and mention of the grant of the patent: **21.08.2019**
(21) Application number: 14724971.8
(22) Date of filing: 18.04.2014
(51) Int. Cl.: A61M 25/09

(54) **GUIDE WIRE INSERTION AID**
FÜHRUNGSDRAHTEINFÜHRHILFE
DISPOSITIF D'AIDE À L'INSERTION DE FIL-GUIDE

(30) Priority: 25.04.2013 JP 2013091883
(43) Date of publication of application: 02.03.2016
(73) Proprietor: Covidien LP, Mansfield, MA 02048 (US)
(72) Inventor: KAZUHIRO, Koike, Tokyo, 158-8615 (JP)
(74) Representative: Gray, James
(86) International application number: PCT/US2014/034622
(87) International publication number: WO 2014/176124

(56) References cited:
- EP-A1- 2 433 670
- WO-A1-01/41860
- WO-A1-2007/124076
- US-A- 6 059 484
- US-B1- 6 533 772

## Description

### FIELD OF THE INVENTION

The present invention relates to a guide wire insertion aid for inserting a guide wire into a cavity of a medical instrument.

### BACKGROUND OF THE DISCLOSURE

Conventionally, there are times when a guide wire is desired to be inserted into a cavity of a medical instrument when performing a medical procedure. The guide wire is normally contained in an inserted state in a tube shaped wire receptacle (dispenser). Further, a guide wire insertion aid (slider) for aiding the dispensing action of the guide wire is attached to the wire receptacle.

A receptacle supporter (coupling) is provided on a base end side of the conventional guide wire insertion aid. The wire receptacle for containing the guide wire is detachably supported by the receptacle supporter. Meanwhile, a guide wire supporter (cannula insertion part) having a conical and cylindrical shape is provided on the tip end side of the guide wire insertion aid. The guide wire dispensed from the wire receptacle is inserted and supported by the guide wire supporter. A sliding part (guide wire sliding part) that has a sliding surface is disposed between the wire receptacle supporter and the guide wire supporter in the guide wire insertion aid.

The guide wire insertion aid configured after this manner is used according to the following procedures when inserting the guide wire into, for example, a body. First, an operator grips the main body between the fingers of one hand so that the guide wire supporter is on the front side. Next, the operator moves a thumb forward so as to slide along the sliding part to dispense the guide wire forward until it protrudes from the tip end of the guide wire supporter.

Here, the guide wire is normally made of a metal fabrication or of a metal with a plastic covering and most of these have elasticity and facilitate a springy characteristic. This type of guide wire may spring out from the guide wire insertion aid or the receiving tool or rotate when applying vibration in a disposed state on the guide wire insertion aid.

Therefore, with the conventional guide wire insertion aid, a sliding part having a smoothly curved bridge shape is provided to suppress the guide wire from springing out when vibration is applied. When doing so, the sliding part raises the guide wire upward that sticks out from the receptacle supporter and applies a slide resistance force by the bridge shape to add constraint to the free movement of the wire. Therefore, the guide wire can be suppressed from springing out when vibration is applied to the guide wire insertion aid.

### PRIOR ART

US 6,059,484 describes an apparatus comprising an introduction aid and a connection element, wherein the apparatus stretches one end of a catheter guide wire and secures the catheter guide wire in a dispenser tube.

EP 2,433,670 describes a guidewire insertion aid for feeding out a guidewire inserted in a housing, to insert the guidewire into the lumen of a piece of medical equipment. US 6,533,772 describes a guidewire torque device, comprising an elongate tubular body with an interior longitudinal channel and a clamp for gripping and ungripping a guide wire extending through the channel.

WO 2007/124076 describes a guidewire handling device comprising a device body, having a guidewire passage sized to receive a guidewire, a movable clamp, a visible marker, and a contoured surface allowing torque applied to the device to be dependent on location of application.

WO 01/41860 describes a manual control device for a surgical guide, comprising a body with a duct through which the surgical guide passes, and an axial blocking mechanism.

### SUMMARY OF THE DISCLOSURE

In the first aspect of the present invention, a guide wire insertion aid, as disclosed in claim 1, that is an aid for dispensing a guide wire inserted in a wire receptacle and inserting the guide wire into a cavity of a medical instrument comprises a main body portion having proximal and distal ends, a wire receptacle supporter at the proximal end of the main body portion configured to support the wire receptacle, and a guide wire supporter at the distal end of the main body portion having a tip end with an opening. The guide wire supporter is configured to support the guide wire that is inserted and dispensed from the wire receptacle. A holding member may extend distally from the wire receptacle support toward the guide wire supporter. A clamping member is secured to the main body portion. The clamping member may be adjacent the distal end of the main body portion and extends distally to a terminal end margin of the clamping member that is generally adjacent the proximal end of the main body portion. The clamping member is movable relative to the main body portion between a clamping position, in which the terminal end margin of the clamping member is configured to engage the guide wire and clamp the guide wire between the terminal end of the clamping member and the holding member, and a non-clamping position, in which the terminal end margin of the clamping member is configured to disengage the guide wire and allow free movement of the guide wire. The clamping member is resiliently flexible along its length and resiliently biased to the clamping position, and the guide wire insertion and is configured to allow an operator to dispense the guide wire in either the distal direction or the proximal direction using a sliding operation with a finger while the guide wire is in the clamping position.

Other objects and features will be in part apparent and in part pointed out hereinafter.

### BRIEF DESCRIPTION OF THE DRAWINGS

FIG. 1 is a front view of the guide wire insertion aid attached to the wire receptacle in a specific first embodiment of the present invention;
FIG. 2 is an enlarged front view of the guide wire insertion aid of the first embodiment, illustrating a clamping member in a clamping position;
FIG. 3 is similar to FIG. 2, except illustrating the clamping member in a non-clamping position;
FIG. 4 is similar to FIG. 3, except illustrating the clamping member in a locked, non-clamping position;
FIG. 5 is an enlarged front view of a guide wire insertion aid of a second embodiment, illustrating a clamping member in a clamping position;
FIG. 6 is similar to FIG. 5, except illustrating the clamping member in a locked, non-clamping position; and
FIG. 7 is an enlarged perspective view of main components of a holding member of the guide wire insertion aid of the second embodiment.

Corresponding reference characters indicate corresponding parts throughout the drawings.

### DESCRIPTION OF THE PREFERRED EMBODIMENTS

With the conventional guide wire insertion aid, a sliding resistance force is applied to the guide wire from the sliding part having a bridge shape and the free movement of the guide wire is constrained, however, when a vibration is applied over a long period of time, such as when transporting a product, or when there is a large impact, the resistance force is insufficient and the guide wire moves freely. As a result, the guide wire springs off from the guide wire insertion aid or rotates and the guide wire is not collected at a predetermined position at the time of use. Therefore, depending on the operator, there may be a need to return it to its original position at the time of use and thereby it has poor usability.

Further, in the conventional guide wire insertion aid, there are those that suppress movement of the guide wire by mounting a separate and independent part such as, for example, a rubber ring onto the guide wire insertion aid. With this type of guide wire insertion aid, the free movement of the guide wire is effectively suppressed, but there is a problem with manufacturing cost in that the part count of a product is increased thereby increasing the manufacturing cost.

Furthermore, a guide wire insertion aid is desired that has a simple operation to enable suppression of the free movement of the guide wire and that has excellent operability to easily perform the sliding operation of the guide wire better than that of the conventional guide wire insertion aid.

In light of the problems described above, an object of at least one embodiment of the present invention is to provide a guide wire insertion aid having a structure that suppresses manufacturing cost, that can effectively suppress free movement of the guide wire when a vibration or impact is applied, and that has excellent operability.

In order to achieve the above objective, a characteristic of the guide wire insertion aid according to the present invention is that an aid for dispensing a guide wire inserted in a wire receptacle and inserting the guide wire into a cavity of a medical instrument includes, as described in claim 1, a main body portion having proximal and distal ends; a wire receptacle supporter at the proximal end of the main body portion configured to support the wire receptacle; a guide wire supporter at the distal end of the main body portion having a tip end with an open cylindrical shape, wherein the guide wire supporter is configured to support the guide wire that is inserted and dispensed from the wire receptacle; a holding member extending distally from the wire receptacle support toward the guide wire supporter; and a clamping member connected to the main body portion adjacent the distal end of the main body portion and extending distally to a terminal end margin of the clamping member that is generally adjacent the proximal end of the main body portion, wherein the clamping member is movable relative to the main body portion between a clamping position, in which the terminal end margin of the clamping member is configured to engage the guide wire and clamp the guide wire between the terminal end of the clamping member and the holding member, and a non-clamping position, in which the terminal end margin of the clamping member is configured to disengage the guide wire and allow free movement of the guide wire, wherein the clamping wire is resiliently flexible along its length and resiliently biased to the clamping position, and wherein the guide wire insertion aid is configured to allow an operator to dispense the guide wire in either the distal direction or the proximal direction using a sliding operation with a finger while the guide wire is in the clamping position.

Because the clamping member of the guide wire insertion tool has flexibility, applying pressure by pressing the clamping member with a finger bends the location coupled to the main body portion to a base point while storing elasticity. Further, the bent clamping member returns to its original form when releasing the applied pressure due to the elasticity. According to this operation, the terminal end margin of the clamping member is made to swing. Furthermore, the terminal end margin of the clamping member and the holding member are configured so that the guide wire can be clamped when disposed therebetween, and is clamped by forces from two directions of the terminal end margin of the clamping member and the holding member. In this case, it is preferred that the guide wire be clamped when the pressure applied on the clamping member is completely released. The guide wire is clamped and released by the swinging action of the clamping member.

When the guide wire is disposed between the terminal end margin of the clamping member and the holding member by this clamping force, the free movement of the guide wire is effectively suppressed. Further, because a clamping force is applied from not only a resisting force from one direction but from two directions on the guide wire, a large slide resisting force is applied by the guide wire. Therefore, the guide wire is suppressed from springing out from the guide wire insertion aid or rotating even when a vibration or impact is applied to the guide wire. In doing so, the guide wire disposed in advance in the guide wire insertion aid does not move at the time of use and stays in a predetermined position in the guide wire insertion aid.

Accordingly, the need to return a moved guide wire to its original position is eliminated, and the usability for the operator is improved. Further, an operator, by swinging the clamping member, can easily suppress and release the free movement of the guide wire. Therefore, operability by a user is improved. Note that the configuration allows an operator to dispense the guide wire in either the distal direction or the proximal direction using a sliding operation with a finger while the guide wire is clamped.

In addition, with the guide wire insertion aid, the guide wire is clamped by the terminal end margin of the clamping member and the holding member provided on the guide wire insertion aid. Therefore, the guide wire can be clamped in the guide wire insertion aid without increasing the number of parts and thereby can minimize increases in manufacturing cost. In this case, it is preferred that the guide wire insertion aid is formed so that the clamping member and the holding member are made of the same material as the main body portion and are molded in one piece. Note that clamp means here that the guide wire is interposed by the clamping member and the holding member in a state that makes free movement difficult, and it does not mean that there is always a firm grip. A grip is preferred using a moderate force that will not damage the guide wire by clamping. Further, the terminal end margin of the clamping member and the holding member means not only the terminal end margin of the clamping member and the holding member but the periphery of the terminal end margin of the clamping member and the periphery of the holding member, respectively.

Additionally, with this guide wire insertion aid, not only is the guide wire made to slide by sliding a finger on the sliding surface but the clamping member can also be made to swing. Therefore, when sliding the guide wire, the clamping member is pressed by a finger and, at the same time, swings so as to bounce by the amount of force of the finger. For example, before dispensing the guide wire, applying a strong finger force to the clamping member greatly bends the clamping member. Further, a finger force applied to the clamping member while dispensing the guide wire gradually weakens and the bend in the clamping member moves to return to its original form. The clamping member can be made to swing by repeating this operation. An operator utilizing this swinging action can dispense the guide wire with a proper sliding operation rhythm, and thus the operability by a user is further improved. Note that the clamping member is preferably structured so as to move deeply into the main body portion side by the applied pressure.

Furthermore, another structural characteristic of the guide wire insertion aid according to one embodiment is that the terminal end margin of the clamping member and the holding member are formed to mutually interfit, and a groove for receiving the guide wire is provided in either or both the terminal end margin of the clamping member or the holding member.

According to this, the terminal end margin of the clamping member and the holding member both interfit by, for example, a pinching operation with the fingers. Further, because a groove is provided on either or both the terminal end margin of the clamping member or the holding member, the guide wire can be positioned in the groove. By this, when the guide wire that is disposed between the clamping member and the holding member that are interfit, the guide wire can be held to be fit in the groove. Then, the guide wire is held clamped by the terminal end margin of the clamping member and the holding member. At this time, because the terminal end margin of the clamping member and the holding member are interfit, the clamping of the guide wire does not release even if there is a force, not shown in the figure, applied to the clamping member. Further, by positioning the guide wire in the groove, deformation or damage to the guide wire clamped by the terminal end margin of the clamping member and the holding member is reduced. Therefore, even if when a strong vibration or impact is applied during transport or the like of a product, or when the clamping member is susceptible to the application of a force, because the fit between the clamping member and the holding member is maintained and the guide wire continues to be clamped by the clamping member and the holding member, and springing out or rotation of the guide wire can be more reliably suppressed. Furthermore, this configuration can achieve suppression of manufacturing costs without increasing the part count. Note that when the clamping member and the holding member are interfit, it is preferred that they face each other.

Further, another structural characteristic of the guide wire insertion aid according to one embodiment of the present invention is a latching mechanism. A hook is provided on the clamping member to protrude to the main body portion side, and a keeper is provided that latches with the hook such that the terminal end margin of the clamping member and the holding member are separated.

By this, the hook and the keeper can latch together, and when latched, the clamping member is releasably held or fastened to the main body portion in a second position. Accordingly, the clamping member no longer swings, and the terminal end margin of the clamping member and the holding member are maintained in a separated state. Therefore, when the guide wire is disposed in the guide wire insertion aid, the guide wire is held in a manner that allows free movement. In this way, when the operator slides the guide wire, the configuration can be selected in which the guide wire is held such that it can already freely move, and operability of the guide wire for the operator is further improved. Further, releasing the latching of the hook and the keeper allows the clamping member to simply return to its original swinging state (i.e., the first position).

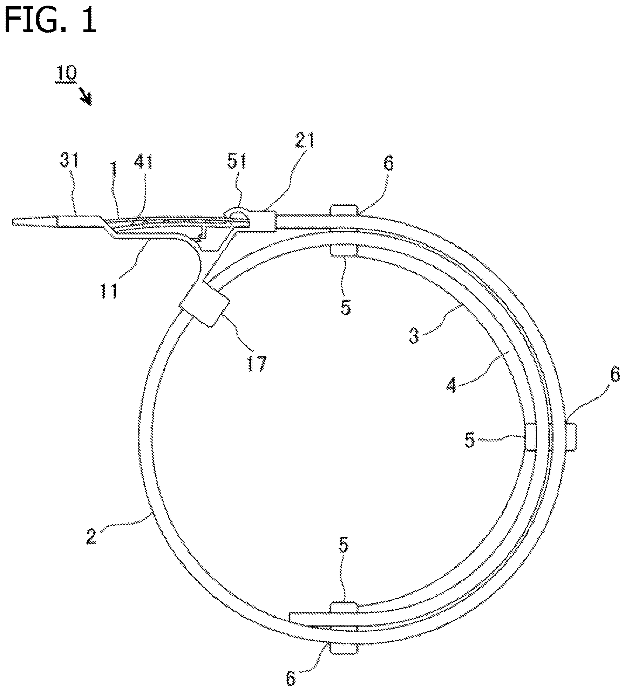

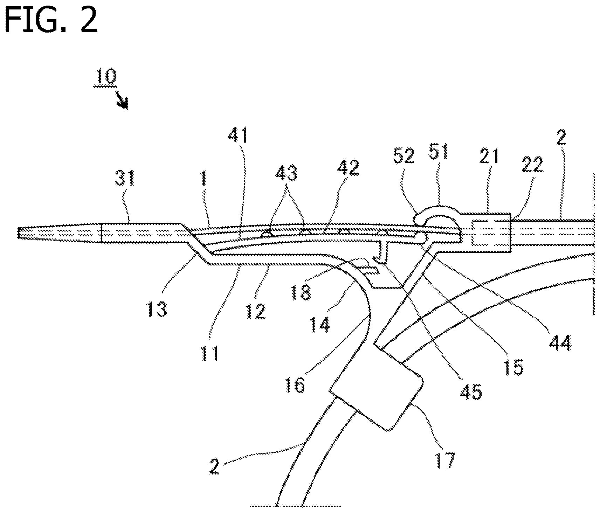

Referring now to the Figures, FIG. 1 is a front view illustrating that the guide wire insertion aid is attached to the wire receptacle in a specific first embodiment of the present invention. FIGS. 2 to 4 are magnified front views illustrating the guide wire insertion aid of the first embodiment. A description will be given hereafter of a guide wire insertion aid 10 in a specific first embodiment of the present invention based on FIGS. 1 to 4. Note that, for convenience in explanation, the right side in the drawing is referred to as "right" and the left side is referred to as "left." Further, the top side in the drawing will be referred to as "top" or "upper" and the bottom side will be referred to as "bottom" or "lower." Further, the up and down direction on the paper surface in the drawing will be referred to as the "width direction." In addition, the end of the direction in which the guide wire 1 in the guide wire insertion aid 10 proceeds from the guide wire insertion aid 10 will be referred to as the "distal end" or "distal direction," and the end of the direction that the guide wire 1 returns will be referred to as the "proximal end" or "proximal direction."

FIG. 1 illustrates the guide wire insertion aid 10 and a wire receptacle 2 in this embodiment. The wire receptacle 2 is composed of a tube made of a synthetic resin partially double wrapped, and the guide wire 1 is inserted and contained in the interior thereof. A holder 3 made of a synthetic resin functions to hold a roll shaped wire receptacle 2. Specifically, the holder 3 of this embodiment is provided with a base part 4 having a substantial C shape and a locking part 5 provided on both ends and the center section of the base part 4. A plurality of locking grooves 6 are formed on each locking part 5, and the wire receptacle 2 is fit and locked with the locking grooves 6. Here, a metal wire or a resin covered wire having a wire made of metal covered by plastic is used as the guide wire 1 in this embodiment. A depth mark is placed at each predetermined length on the wire surface. The wire tip end is composed of a J type curved in a J shape, an angled shape, and a straight shape.

As illustrated in FIGS. 1 to 4, the guide wire insertion aid 10 of this embodiment is made of a synthetic resin (for example, polypropylene) and is provided with a main body portion 11 in the center. The main body portion 11 has an overall long and thin shape that extends in the lateral direction. A receptacle supporter 21 is integrally provided on the proximal side of the main body portion 11 and is coupled to the proximal side of the main body portion 11 at the left lower side of the receptacle supporter 21. The receptacle supporter 21 is formed in a substantially square shape and is provided with a fitting hole 22 having a cylindrical shape that penetrates in the lateral direction. One end of the wire receptacle 2 is detachably fit to the fitting hole 22 that opens at the proximal surface of the receptacle supporter 21. The wire receptacle 2 is supported by the receptacle supporter 21 by this fitting.

Meanwhile, a guide wire supporter 31 is integrally provided on the distal side of the main body portion 11 and is coupled to the distal side of the main body portion 11 at the right lower side of the guide wire supporter 31. The guide wire supporter 31 tapers distally as it approaches the tip end, and at the tip end it opens to form a hollow cylindrical shape. The guide wire 1 dispensed from the wire receptacle 2 side is inserted inside the guide wire supporter 31. As a result, the guide wire 1 is supported by the guide wire supporter 31.

A flat part 12 formed in a horizontal plate like shape that extends in the lateral direction is provided on the main body portion 11. A first linking part 13 having an integral and continuous plate like shape on the proximal side of the guide wire supporter 31 is provided on the distal side of the main body portion 11 and extends to the upper left from the tip end of the flat part 12. A curved part 14 having a curved plate like shape that extends in an arc downward extends from the proximal side of the flat part 12. Further, a second linking part 15 having a plate like shape that extends bending sharply downward to the left after being horizontally extended to the tip end aside from the bottom part of the distal side of the receptacle supporter 21, is provided on the distal side on the main body portion 11.

On the lower side of the main body portion 11, a one-piece abutment 16 is formed where the curved part 14 and the second linking part 15 come together. A cross-sectional substantially U shaped securing part 17 is integrally provided on the lower end of the abutment 16. The securing part 17 holds or grips the wire receptacle 2 by latching with the outer peripheral surface of the wire receptacle 2. As a result, the guide wire insertion aid 10 and the wire receptacle 2 are mutually difficult to displace. Further, when an operator grips the guide wire insertion aid 10 in one hand, a finger other than the thumb can be used to hook in the curved part 14. Therefore, the guide wire insertion aid 10 can be easily gripped by an operator.

A plate shaped clamping member 41 is integrally provided with the main body portion 11 between the receptacle supporter 21 and the guide wire supporter 31. The clamping member 41 is formed so as to allow the guide wire 1 to slide between the receptacle supporter 21 and the guide wire supporter 31. Further, the clamping member 41 is disposed above the flat part 12. The clamping member 41 is made of a member having flexibility (for example, polypropylene) and has a supple characteristic for storing an elastic force when pressed. The upper surface on the clamping member 41 configures a sliding surface 42 where the guide wire 1 moves while contacted along its linear axis direction.

The tip part of the distal side of the clamping member 41 is integrally coupled to the proximal side of the midsection of the first linking part 13. Further, the clamping member 41 extends to the proximal side along a direction that is nearly parallel to the main body portion 11 positioned on the lower side. Meanwhile, the proximal side of the clamping member 41 is fixed. By this, the clamping member 41 is configured so that the terminal end margin can swing based on the distal side of the swinging part 41. Further, as illustrated in FIG. 2, when in a natural state having no pressure applied to the clamping member 41, the clamping member 41 has elasticity and has an upward working force on the terminal end margin of the clamping member 41. According to this configuration, the guide wire 1 contacts the holding member 51 to be described below when not disposed on the clamping member 41.

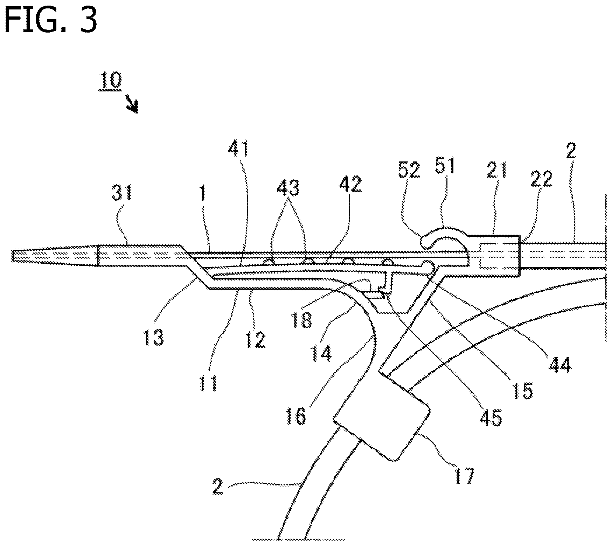

As illustrated in FIG. 3, when pressure is applied to the sliding surface 42, the terminal end margin of the clamping member 41 greatly flexes to move downward on the basis of the distal side of the clamping member 41 being coupled to the main body portion 11. Moreover, when the pressure is relaxed, the flexing of the clamping member 41 moves in the direction to return to its origin due to the elasticity stored in the clamping member 41, and when the pressure is released, the clamping member 41 returns to its original position. The clamping member 41 can be made to swing by repeating the applying and releasing of pressure on the clamping member 41.

Further, a plurality of ribs 43 are provided on the clamping member 41 and are formed along the width direction of the sliding surface 42 in a cross-sectional substantially semicircular shape on the upper surface. The guide wire 1 arranged on the sliding service 42 is raised slightly upward by these ribs 43. Raising the guide wire 1 upward functions to improve the operability for the operator. These ribs 43 are preferably disposed in equal spacing on the sliding surface 42. Further, as long as each of the ribs 43 can contact the guide wire 1, they may be formed on only a portion of the width direction of the sliding surface 42.

The terminal end margin of the clamping member 41 (hereinafter referred to as swinging end 44) having a rib shape formed along the width direction of the sliding surface 42 is provided on the proximal side of the clamping member 41. The top surface of the swinging end 44 has a planar shape. Further, the swinging end 44 swings together with the clamping member 41. Note that in this embodiment, the swinging end 44 is provided at the terminal end of the clamping member 41, but it may be provided near the distal side of the clamping member 41.

A holding member 51 having a plate like shape curved in a cross-sectional substantially semicircular shape that extends toward the terminal end margin of the clamping member 41 is integrally provided with the receptacle supporter 21 on the top part of the distal side of the receptacle supporter 21. The distal end of the holding member 51 (hereinafter referred to as the holding end 52) holding member has a flat surface formed along the width direction of the holding member 51 facing the downward side. As illustrated in FIG. 2, when the clamping member 41 is not pressed, the guide wire 1 is clamped by the swinging end 44 and the holding end 52 when the guide wire 1 is not disposed on the clamping member 41. At this time, because the swinging end 44 and the holding end 52 are both formed having a flat surface, the guide wire 1 is easily interposed and gripped by both flat surfaces so as to suppress any free movement. Further, clamping the guide wire 1 by the flat surfaces of the swinging end 44 and the holding end 52 allows various external diameter sizes of the guide wire 1 to be similarly clamped by the guide wire insertion aid 10.

Further, as illustrated in FIG. 3, when pressure is applied to the clamping member 41, the swinging end 44 and the holding end 52 separate to release clamping of the guide wire 1. By this, the guide wire 1 can move freely. Further, when the guide wire 1 is not disposed in the clamping member 41, and pressure is not being applied to the clamping member 41, the swinging end 44 and the holding end 52 mutually contact on their respective flat surfaces.

On the lower part slightly closer to the proximal side from the midsection of the clamping member 41, a hook 45 having a protrusion in the shape of a hook along the width direction on the distal end thereof is integrally provided on the clamping member 41 to protrude to the main body portion 11 side. The lower end of the hook45 has a semi-sagittal shape and is formed having a substantially right angle hook facing the distal side with a slanted surface facing the curved part 14 side. Further, the keeper18 is integrally formed on the curved part 14 along the width direction so as to protrude nearly horizontally to the proximal side. The keeper 18 is formed having a slanted surface where the protruding right end surface slants facing downwards. Note that, the hook45 is preferably provided in a position of one fourth to one third from the proximal side of the overall length of the clamping member 41. Additionally, the shape of the hook45 may be another shape of hook other than a right angle hook as long as it can latch with the keeper 18.

As illustrated in FIG. 3, with the hook45 and the keeper 18, when a strong pressure is applied to the clamping member 41 and the clamping member 41 has sunken deeply downward, the swinging end 44 and the holding end 52 are separated and the slanted surface of the hook45 contacts the slanted surface of the keeper 18. The downward movement of the clamping member 41 by this pressing operation is stopped. At this time, a state is kept where the guide wire 1 can move freely.

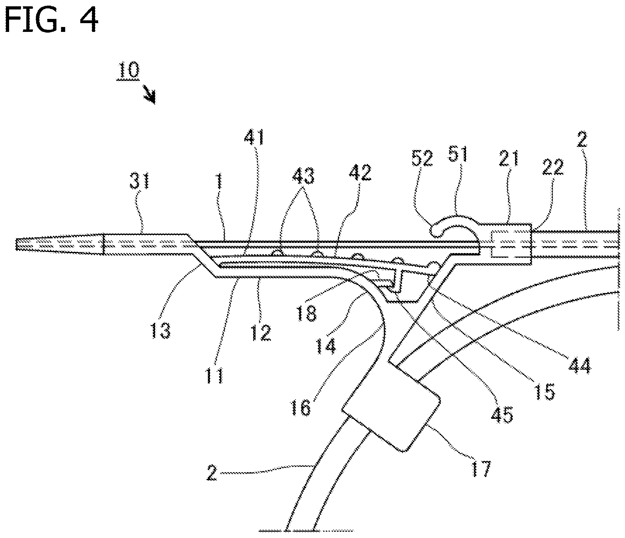

Further, as illustrated in FIG. 4, applying stronger pressure to the clamping member 41 allows the hook 45 to flex to go over the tip of the keeper 18 so that the hook 45 latches with the keeper 18. This latching creates a state in which the clamping member 41 does not move and cannot swing. The swinging end 44 and the holding end 52 are separated when in this state, and therefore, a state is kept where the guide wire 1 can move freely. Applying an upward force to the clamping member 41 releases the latching of the hook 45 and the keeper 18 so that the clamping member 41 can once again swing.

A description will be given hereafter of a catheter insertion method utilizing the Guide wire insertion aid 10, which is to say, a method for inserting the guide wire 1 into a cavity of a medical instrument such as a catheter by dispensing through the wire receptacle 2 using the guide wire insertion aid in 10.

After once again disinfecting the skin around the insertion area of the patient, a local anesthesia is administered to the insertion location. Next, a blood vessel is tapped using an intravascular cannula with a mounted syringe barrel. After verifying a reverse flow of blood, the metal syringe of the intravascular cannula is retained and only a plastic cannula proceeds into the blood vessel and remains.

Next, using the guide wire insertion aid 10, the guide wire 1 passes through the indwelling cannula and the guide wire 1 inserts into the blood vessel.

In this case, fit one end of the wire receptacle 2 that contains the guide wire 1 into the fitting hole 22 of the receptacle supporter 21, and newly fill the inside of the wire receptacle 2 with a heparin added physiological saline solution injected from the other end. Further, as illustrated in FIG. 2, without pressing the clamping member 41, clamp the guide wire 1 with the swinging end 44 and the holding end 52 to create a state where the guide wire 1 is secured and cannot dispense naturally. Then, verify that the distal of the guide wire 1 is in the guide wire supporter 31.

Here, an operator grips the main body 21 between the fingers of one hand so that the guide wire supporter 31 is on the distal side. Next, insert the distal of the guide wire supporter 31 into the plastic cannula. Dispense the guide wire 1. Specifically, press down on the clamping member 41 applying the pad of the thumb to the sliding surface 42, and slide the pad of the thumb along the sliding surface 42 while gripping the guide wire 1 with the pad of the thumb. When doing so, the guide wire 1 is dispensed from the distal side. Further, similarly, the guide wire 1 can be operated to retract into the proximal side.

With this retracting operation, the clamping member 41 swings on the basis of distal side being coupled to the main body portion 11. In other words, as illustrated in FIG. 3, before dispensing the guide wire 1, the clamping member 41 is pressed by the thumb downward applying a force and sinks downward while flexing (i.e., to a second position). Further, when dispensing the guide wire 1 distally, the force that was applied to downward on the clamping member 41 is released upward to relax the pressure. In conjunction with this, the clamping member 41 rebounds due to the elastic force of the clamping member 41, and the flexing of the clamping member 41 returns to its original position (i.e., the first position). When repeating this type of dispensing operation, the clamping member 41 rhythmically swings as if bouncing, and the guide wire 1 is successively dispensed forward. In addition to this, when the operator presses the guide wire 1 with the surface of the finger during the sliding operation of the guide wire 1, the guide wire 1 slightly flexes between the plurality of ribs 43 generating a counterforce from the guide wire 1. This counterforce improves contact with the guide wire 1 by the finger of the operator and aids the sliding operation of the guide wire 1 by the operator.

With this process for the dispensing operation, as long as pressure is applied to the clamping member 41, the guide wire 1 will not be clamped by the swinging end 44 and the holding end 52. Meanwhile, when releasing the pressure applied to the clamping member 41, the swinging end 44 and the holding end 52 immediately clamp the guide wire 1 so as to suppress free movement of the guide wire 1.

Prior to the dispensing operation or in the process thereof, as illustrated in FIG. 4, the operator may flex the clamping member 41 downward by applying a large pressure to thereby latch the hook 45 and the keeper 18. When doing so, the clamping member 41 is fixed to the main body portion 11, and the clamping member 41 is unable to swing. In this case, a state is held in which the guide wire 1 is not clamped by the clamping end 44 and the holding end 52, and the free movement of the guide wire 1 is continuously allowed.

As a result of performing the above dispensing operation, the guide wire 1 is inserted in the blood vessel via the cannula. When performing this type of operation, it is best to verify that the guide wire 1 is in the target position using an x-ray image.

After completing insertion of the guide wire 1, remove the cannula and insert a small surgical incision at the insertion site using a skin incision knife. Here, proceeding to press a dilator along the guide wire 1 sufficiently spreads the subcutaneous tissue and the blood vessel entry. Remove the dilator after spreading. Next, slowly insert the catheter along the guide wire 1. At this time, proceed to insert the catheter to the target indwelling position while verifying the depth mark. Once the catheter is confirmed to reside in the target position, remove the guide wire 1. Next, remove the air in the cavity of the catheter using a standard method and flush the inside of the cavity with a physiological saline solution or a heparin added physiological saline solution to prevent clotting. At this time, verification under an x-ray image is preferred for verifying that the catheter tip end is in the target position and that the catheter is not depicting a loop inside the body. Subsequently, secure the catheter with a catheter fixture and a suture needle with thread in the series of operations for catheter insertion is completed.

According to the guide wire insertion aid 10 of this embodiment as described above, when the guide wire 1 is disposed in the guide wire insertion aid 10, the guide wire 1 is clamped between the clamping end 44 and the holding end 52 so as to suppress free movement of the guide wire 1. Therefore, the guide wire 1 is suppressed from springing out from the guide wire insertion aid 10 or rotating even when a vibration or impact is applied to the guide wire.

Further, according to the guide wire insertion aid 10 of this embodiment, the guide wire 1 is clamped by the clamping end 44, which is one part of the guide wire insertion aid 10, and the holding end 52. By this, there is no need to increase the part count in order to provide a function for holding the guide wire 1. Therefore, an increase in manufacturing costs can be suppressed while providing a function for suppressing the free movement of the guide wire 1.

Further, according to the guide wire insertion aid 10 of this embodiment, fixing and releasing of the guide wire 1 that is clamped by the clamping end 44 and the holding end 52 is easily achieved by applying pressure to the clamping member 41. In addition, by creating a favorable rhythm to the sliding operation of the guide wire 1 by an operator using the clamping member 41 to aid the operation of a rhythmical swing, excellent operability can be achieved. Moreover, because an operator can utilize the counterforce provided by the flexing of the guide wire 1 created between a plurality of ribs 43 to perform the sliding operation of the guide wire 1, even further excellent operability can be achieved.

Furthermore, according to the guide wire insertion aid 10 of this embodiment, the hook45 latches with the keeper 18 to thereby stop swinging of the clamping member 41 and thus can fix the clamping member 41. Therefore, according to selection by the operator, a sliding operation of the guide wire 41 can be performed by a clamping member 41 that does not swing.

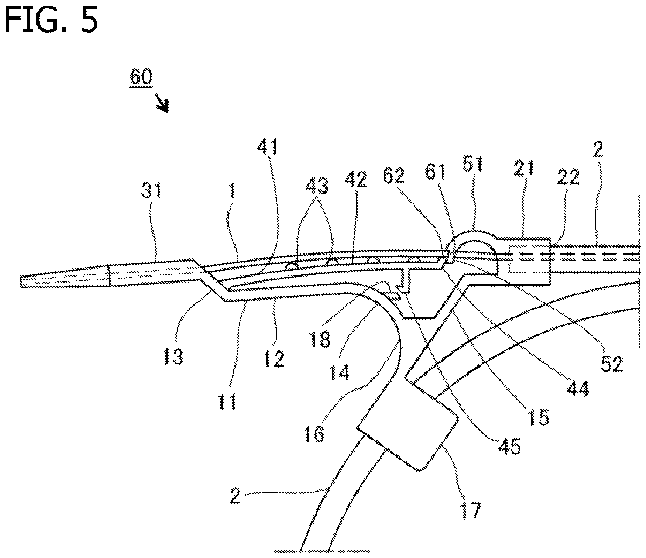

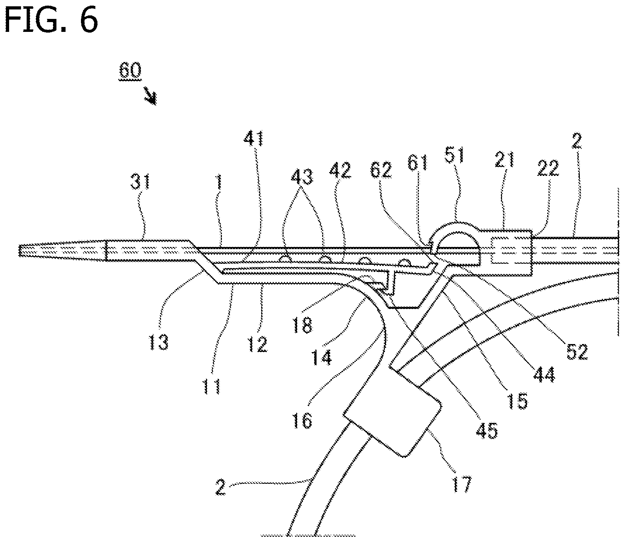

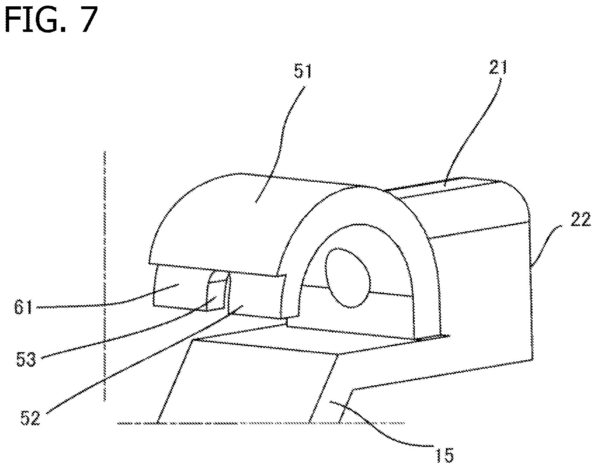

FIGS. 5 and 6 are magnified front views illustrating the guide wire insertion aid of the second embodiment. FIG. 7 is a magnified perspective view of the main parts illustrating the holding member of the guide wire insertion aid of the second embodiment. A description will be given hereafter of a guide wire insertion aid 60 in a specific second embodiment based on FIGS. 5 and 6.

As illustrated in FIGS. 5 and 6, the guide wire insertion aid 60 of this embodiment has a main body portion 11, a receptacle supporter 21, a guide wire supporter 31, a clamping member 41, a holding member 51, and a keeper 18, and other than the shape of the swinging end 44 and the holding end 52, it has a similar structure to the guide wire insertion aid 10 described above. Accordingly, the same reference numerals will be applied to the same parts and explanations thereof will be omitted. A step part 61 forming a shape where the distal direction along the width direction is notched on the upper level is provided on the holding end 52. Further, as illustrated in FIG. 7, a substantial inverted U shaped groove 53 is provided in the center of the width direction of the step part 61. The width of this groove 53 is suited to the external shape of the guide wire 1 so as to be positioned for insertion into the groove 53 when the guide wire 1 is clamped by the swinging end 44 and the holding end 52. Further, the groove 53 has a size such that the guide wire 1 does not deform and is not scratched when positioned therein. The surface of the lower side of the step part 61 is formed in a planar shape.

A corner part 62 that projects upward along the width direction of the clamping member 41 and that provides a flat surface on both the upper and proximal side, is provided on the swinging end 44. Further, the upper surface and the proximal side surface of the corner part 62 is suited to the shape of the notch on the upper level of the step part 61. The corner part 62 is incorporated in the notched portion on the upper level of the step part 61, and the step part 61 and the corner part 62 can interfit. As illustrated in FIG. 5, when the step part 61 and the corner part 62 are interfit, and elastic force from the clamping member 41 is applied to the swinging end 44 and an elastic force from the holding member 51 is applied to the holding end 52, respectively, and the swinging end 44 and the holding end 52 are in a firmly pressed state. Therefore, the fit will not easily be released due to vibration or impact.

The upper surface of the corner part 62 can contact the lower surface of the step part 61 that faces downward and may contact even when no pressure is applied to the clamping member 41. Note that the shapes of the swinging end 44 and the holding end 52 are not particularly limited. In this embodiment, a configuration is illustrated in which the step part 61 is provided on the holding end 52, and the corner part 62 is provided on the swinging end 44, but the corner part may be provided on the holding end 52 and the step part may be provided on the swinging end 44. Other shapes than the step part 61 are possible for the swinging end 44, and other shapes than the corner part 62 are possible for the holding end 52 as long as the swinging end 44 and the holding end 52 interfit. Additionally, in this embodiment, the groove 53 is illustrated as being provided on the holding end 52, but the groove may be provided on the swinging end 44. The groove may be provided on both the swinging end 44 and the holding end 52.

When clamping the guide wire 1 using the guide wire insertion aid 60, the guide wire 1 is first arranged in the clamping member 41. In this state, as illustrated in FIG. 6, applying pressure to the clamping member 41 allows the guide wire 1 to freely move. Further, when the guide wire 1 is not inserted in the groove 53, which is to say when the position of the guide wire 1 is not in the groove 53, releasing the pressure applied to the clamping member 41 clamps the guide wire 1 by the swinging end 44 and the holding end 52. In this state, the corner part 62 and the step part 61 of the holding member 51 are not yet interfit.

Next, the guide wire 1 is disposed so as to insert into the groove 53. Also, as illustrated in FIG. 5, the step part 61 and the corner part 62 are interfit. In other words, the clamping member 41 is pinched by fingers and pressed while bending to the distal side from the above state and interfit after the corner part 62 goes past the tip of the step part 61. When doing so, the guide wire 1 is held by the corner part 62 while fit in the groove 53 and is firmly clamped. At that time, light pressure is applied to the clamping member 41 such that the interfitting of the step part 61 and the corner part 62 does not come apart even if an impact or vibration is applied, and the guide wire 1 continues to be clamped. To release the interfitting, the reverse of the above operation is required.

When the clamping member 41 and the holding member 51 are interfit in this manner when the guide wire 1 is disposed in the groove 53, the guide wire 1 is firmly held fit in the groove 53. The guide wire 1 is unlikely to be deformed or scratched by fitting and clamping the guide wire 1 in the groove 53. Therefore, when it is necessary that the guide wire 1 is held over a long period of time such as during transport of a product, a firm hold is possible by the guide wire insertion aid 60, and springing out or rotating of the guide wire 1 can be more securely suppressed. Furthermore, this configuration can achieve suppression of manufacturing costs without increasing the part count.

Further, the guide wire insertion aid according to the present invention is not limited to the embodiments described above and may be suitably modified within the technical scope of the present invention. For example, a configuration having a look part and a keeper was given in the embodiment described above, but this configuration may be omitted. Further, the look part and that the keeper as well as the corner part and the step part are provided along the width direction, respectively, but this may be formed by a portion in the width direction in an area where these are connected. In addition, a function for latching or interfitting the shapes of the swinging end and the holding end may be provided by the shape illustrated in the embodiments or it may be provided by a different shape.

Having described the invention in detail, it will be apparent that modifications and variations are possible without departing from the scope of the invention defined in the appended claims.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above constructions, products, and methods without departing from the scope of the invention, it is intended that all matter contained in the above description and shown in the accompanying drawings shall be interpreted as illustrative and not in a limiting sense.

## Claims

1. A guide wire insertion aid (10) that is an aid for dispensing a guide wire (1) inserted in a wire receptacle (2) and inserting the guide wire (1) into a cavity of a medical instrument, comprising:
a main body portion (11) having proximal and distal ends;
a wire receptacle supporter (21) at the proximal end of the main body portion (11) configured to support the wire receptacle (2);
a guide wire supporter (31) at the distal end of the main body portion (11) having a tip end with an opening, wherein the guide wire supporter (31) is configured to support the guide wire (1) that is inserted and dispensed from the wire receptacle (2);
a holding member (51); and
a clamping member (41) secured to the main body portion (11), wherein the clamping member (41) is movable relative to the main body portion (11) between a clamping position, in which the terminal end margin of the clamping member (41) is configured to engage the guide wire (1) to clamp the guide wire between the terminal end of the clamping member (41) and the holding member (51), and a non-clamping position, in which the terminal end margin (44) of the clamping member (41) is configured to disengage the guide wire (1) and allow free movement of the guide wire (1),
**characterized in that** the clamping member (41) is resiliently flexible along its length and resiliently biased to the clamping position, and
wherein the guide wire insertion aid (10) is configured to allow an operator to dispense the guide wire (1) in either the distal direction or the proximal direction using a sliding operation with a finger while the guide wire (1) is in the clamping position.

2. The guide wire insertion aid (10) according to claim 1, wherein the holding member (51) is secured to and extends distally from the wire receptacle support (21) toward the guide wire supporter (31), and wherein the clamping member (41) is secured to the main body portion (11) adjacent the distal end of the main body portion (11) and extends distally to a terminal end margin of the clamping member (41) that is generally adjacent the proximal end of the main body portion (11).

3. The guide wire insertion aid (10) according to claim 1, wherein the clamping member (41) has a sliding surface along its length configured to contact the guide wire (1) when the clamping member (41) is in the clamping position.

4. The guide wire insertion aid (10) according to claim 3, wherein the sliding surface includes a plurality of ribs configured to contact the guide wire (1) when the clamping member (41) is in the clamping position.

5. The guide wire insertion aid (10) according to claim 1, wherein the terminal end margin of the clamping member (41) and the holding member (51) are formed to mutually interfit, wherein at least one of the terminal end margin of the clamping member (41) and the holding member (51) defines a groove section for receiving the guide wire (1) therein when the clamping member (41) is in the clamping position.

6. The guide wire insertion aid (10) according to claim 1, further comprising a latching mechanism (15) configured to releasably fasten the terminal end margin (44) of the clamping member (41) to the main body portion (11) when the clamping member (41) is in the non-clamping position.

7. The guide wire insertion aid according to claim 6, wherein the latching mechanism (45) includes a keeper (18) on the main body portion, and a latch on the clamping member (41) configured to releasably engage the keeper (18) to releasably fasten the terminal end margin of the clamping member (41) to the main body portion (11).

8. The guide wire insertion aid (10) according to claim 1, further comprising a securing part (17) on the main body portion (11), wherein the securing part (17) is configured to grip the wire receptacle (2).

9. The guide wire insertion aid (10) according to claim 1, in combination with a wire receptacle (2) and a guide wire (1) received in the wire receptacle (2), wherein the wire receptacle (2) is secured to the wire receptacle support (21) of the guide wire insertion aid (10).

10. The guide wire insertion aid (10) in combination with the wire receptacle (2) and the guide wire (1) according to claim 9, wherein the wire receptacle (2) is removably secured to the receptacle support (21) of the guide wire insertion aid (10).

11. The guide wire insertion aid (10) in combination with the wire receptacle (2) and the guide wire (1) according to claim 9, wherein the guide wire insertion aid (10) further comprises a securing part (17) on the main body portion (11), wherein the securing part (17) grips the wire receptacle (2).

## Patentansprüche

1. Führungsdrahteinführhilfe (10), die eine Hilfe zum Ausgeben eines Führungsdrahts (1), der in einen Drahtbehälter (2) eingeführt ist, und Einführen des Führungsdrahts (1) in einen Hohlraum eines medizinischen Instruments ist, umfassend:
einen Hauptkörperabschnitt (11), der ein proximales und distales Ende aufweist;
einen Drahtbehälterträger (21) an dem proximalen Ende des Hauptkörperabschnitts (11), der dazu konfiguriert ist, den Drahtbehälter (2) zu tragen;
einen Führungsdrahtträger (31) an dem distalen Ende des Hauptkörperabschnitts (11), der ein Spitzenende mit einer Öffnung aufweist, wobei der Führungsdrahtträger (31) dazu konfiguriert ist, den Führungsdraht (1) zu tragen, der in den Drahtbehälter (2) eingeführt ist und aus diesem ausgegeben wird;
ein Halteelement (51); und
ein Klemmelement (41), das an dem Hauptkörperabschnitt (11) gesichert ist, wobei das Klemmelement (41) in Bezug auf den Hauptkörperabschnitt (11) zwischen einer Klemmposition, in der der Rand am terminalen Ende des Klemmelements (41) dazu konfiguriert ist, in Eingriff mit dem Führungsdraht (1) zu treten, um den Führungsdraht zwischen dem terminalen Ende des Klemmelements (41) und dem Halteelement (51) einzuklemmen, und einer Nicht-Klemmposition, in der der Rand (44) am terminalen Ende des Klemmelements (41) dazu konfiguriert ist, außer Eingriff mit dem Führungsdraht (1) zu treten und eine freie Bewegung des Führungsdrahts (1) zu ermöglichen, beweglich ist,
**dadurch gekennzeichnet, dass** das Klemmelement (41) entlang seiner Länge biegeelastisch ist und elastisch in die Klemmposition vorgespannt ist, und
wobei die Führungsdrahteinführhilfe (10) dazu konfiguriert ist, zu ermöglichen, dass ein Bediener den Führungsdraht (1) unter Verwendung eines Schiebevorgangs mit einem Finger entweder in der distalen Richtung oder der proximalen Richtung ausgibt, während sich der Führungsdraht (1) in der Klemmposition befindet.

2. Führungsdrahteinführhilfe (10) nach Anspruch 1, wobei das Halteelement (51) an dem Drahtbehälterträger (21) gesichert ist und sich distal davon in Richtung des Führungsdrahtträgers (31) erstreckt und wobei das Klemmelement (41) benachbart zu dem distalen Ende des Hauptkörperabschnitts (11) an dem Hauptkörperabschnitt (11) gesichert ist und sich distal zu einem Rand am terminalen Ende des Klemmelements (41) erstreckt, der im Allgemeinen benachbart zu dem proximalen Ende des Hauptkörperabschnitts (11) ist.

3. Führungsdrahteinführhilfe (10) nach Anspruch 1, wobei das Klemmelement (41) eine Schiebefläche entlang seiner Länge aufweist, die dazu konfiguriert ist, den Führungsdraht (1) zu berühren, wenn sich das Klemmelement (41) in der Klemmposition befindet.

4. Führungsdrahteinführhilfe (10) nach Anspruch 3, wobei die Schiebefläche eine Vielzahl von Rippen beinhaltet, die dazu konfiguriert ist, den Führungsdraht (1) zu berühren, wenn sich das Klemmelement (41) in der Klemmposition befindet.

5. Führungsdrahteinführhilfe (10) nach Anspruch 1, wobei der Rand am terminalen Ende des Klemmelements (41) und das Halteelement (51) so ausgebildet sind, dass sie ineinanderpassen, wobei mindestens eines von dem Rand am terminalen Ende des Klemmelements (41) und dem Halteelement (51) einen Nutenabschnitt definiert, um den Führungsdraht (1) darin aufzunehmen, wenn sich das Klemmelement (41) in der Klemmposition befindet.

6. Führungsdrahteinführhilfe (10) nach Anspruch 1, ferner umfassend einen Rastmechanismus (15), der dazu konfiguriert ist, den Rand (44) am terminalen Ende des Klemmelements (41) lösbar an dem Hauptkörperabschnitt (11) zu befestigen, wenn sich das Klemmelement (41) in der Nicht-Klemmposition befindet.

7. Führungsdrahteinführhilfe nach Anspruch 6, wobei der Rastmechanismus (45) einen Halter (18) an dem Hauptkörperabschnitt und eine Rastklinke an dem Klemmelement (41) beinhaltet, die dazu konfiguriert ist, den Halter (18) lösbar in Eingriff zu nehmen, um den Rand am terminalen Ende des Klemmelements (41) lösbar an dem Hauptkörperabschnitt (11) zu befestigen.

8. Führungsdrahteinführhilfe (10) nach Anspruch 1, ferner umfassend ein Sicherungsteil (17) an dem Hauptkörperabschnitt (11), wobei das Sicherungsteil (17) dazu konfiguriert ist, den Drahtbehälter (2) zu greifen.

9. Führungsdrahteinführhilfe (10) nach Anspruch 1 in Kombination mit einem Drahtbehälter (2) und einem Führungsdraht (1), der in den Drahtbehälter (2) aufgenommen ist, wobei der Drahtbehälter (2) an dem Drahtbehälterträger (21) der Führungsdrahteinführhilfe (10) gesichert ist.

10. Führungsdrahteinführhilfe (10) in Kombination mit dem Drahtbehälter (2) und dem Führungsdraht (1) nach Anspruch 9, wobei der Drahtbehälter (2) trennbar an dem Behälterträger (21) der Führungsdrahteinführhilfe (10) gesichert ist.

11. Führungsdrahteinführhilfe (10) in Kombination mit dem Drahtbehälter (2) und dem Führungsdraht (1) nach Anspruch 9, wobei die Führungsdrahteinführhilfe (10) ferner ein Sicherungsteil (17) an dem Hauptkörperabschnitt (11) umfasst, wobei das Sicherungsteil (17) den Drahtbehälter (2) greift.

## Revendications

1. Dispositif d'aide à l'insertion de fil-guide (10) qui est un dispositif d'aide pour distribuer un fil-guide (1) inséré dans un réceptacle pour fil (2) et insérer le fil-guide (1) dans une cavité d'un instrument médical, comprenant :
une partie formant corps principal (11) ayant des extrémités proximale et distale ;
un dispositif de support de réceptacle pour fil (21) à l'extrémité proximale de la partie formant corps principal (11) configuré pour soutenir le réceptacle pour fil (2) ;
un dispositif de support de fil-guide (31) à l'extrémité distale de la partie formant corps principal (11) ayant une extrémité formant embout dotée d'une ouverture, dans lequel le dispositif de support de fil-guide (31) est configuré pour soutenir le fil-guide (1) qui est inséré dans le réceptacle pour fil (2) et distribué à partir de celui-ci ;
un élément de retenue (51) ; et
un élément de serrage (41) fixé à la partie formant corps principal (11), dans lequel l'élément de serrage (41) est mobile par rapport à la partie formant corps principal (11) entre une position de serrage, dans laquelle la marge d'extrémité terminale de l'élément de serrage (41) est configurée pour se mettre en prise avec le fil-guide (1) pour serrer le fil-guide entre l'extrémité terminale de l'élément de serrage (41) et l'élément de retenue (51), et une position de non-serrage, dans laquelle la marge d'extrémité terminale (44) de l'élément de serrage (41) est configurée pour se séparer du fil-guide (1) et permettre le libre mouvement du fil-guide (1),
**caractérisé en ce que** l'élément de serrage (41) est flexible élastiquement sur sa longueur et sollicité élastiquement vers la position de serrage, et
dans lequel le dispositif d'aide à l'insertion de fil-guide (10) est configuré pour permettre à un opérateur de distribuer le fil-guide (1) dans la direction distale ou dans la direction proximale à l'aide d'une opération de glissement avec un doigt pendant que le fil-guide (1) est dans la position de serrage.

2. Dispositif d'aide à l'insertion de fil-guide (10) selon la revendication 1, dans lequel l'élément de retenue (51) est fixé au support de réceptacle pour fil (21) et s'étend distalement à partir de celui-ci vers le dispositif de support de fil-guide (31), et dans lequel l'élément de serrage (41) est fixé à la partie formant corps principal (11) de façon adjacente à l'extrémité distale de la partie formant corps principal (11) et s'étend distalement jusqu'à une marge d'extrémité terminale de l'élément de serrage (41) qui est généralement adjacente à l'extrémité proximale de la partie formant corps principal (11).

3. Dispositif d'aide à l'insertion de fil-guide (10) selon la revendication 1, dans lequel l'élément de serrage (41) comporte une surface de glissement sur sa longueur configurée pour être en contact avec le fil-guide (1) lorsque l'élément de serrage (41) est dans la position de serrage.

4. Dispositif d'aide à l'insertion de fil-guide (10) selon la revendication 3, dans lequel la surface de glissement inclut une pluralité de nervures configurées pour être en contact avec le fil-guide (1) lorsque l'élément de serrage (41) est dans la position de serrage.

5. Dispositif d'aide à l'insertion de fil-guide (10) selon la revendication 1, dans lequel la marge d'extrémité terminale de l'élément de serrage (41) et l'élément de retenue (51) sont formés de façon à s'ajuster mutuellement l'une à l'autre, dans lequel au moins l'un de la marge d'extrémité terminale de l'élément de serrage (41) et de l'élément de retenue (51) définit une section formant rainure pour recevoir le fil-guide (1) dans celle-ci lorsque l'élément de serrage (41) est dans la position de serrage.

6. Dispositif d'aide à l'insertion de fil-guide (10) selon la revendication 1, comprenant en outre un mécanisme de verrouillage (15) configuré pour attacher de façon libérable la marge d'extrémité terminale (44) de l'élément de serrage (41) à la partie formant corps principal (11) lorsque l'élément de serrage (41) est dans la position de non-serrage.

7. Dispositif d'aide à l'insertion de fil-guide selon la revendication 6, dans lequel le mécanisme de verrouillage (45) inclut un crochet (18) sur la partie formant corps principal, et un verrou sur l'élément de serrage (41) configuré pour se mettre en prise de façon libérable avec le crochet (18) pour attacher de façon libérable la marge d'extrémité terminale de l'élément de serrage (41) à la partie formant corps principal (11).

8. Dispositif d'aide à l'insertion de fil-guide (10) selon la revendication 1, comprenant en outre une pièce de fixation (17) sur la partie formant corps principal (11), dans lequel la pièce de fixation (17) est configurée pour saisir le réceptacle pour fil (2).

9. Dispositif d'aide à l'insertion de fil-guide (10) selon la revendication 1, en association avec un réceptacle pour fil (2) et un fil-guide (1) reçu dans le réceptacle pour fil (2), dans lequel le réceptacle pour fil (2) est fixé au support de réceptacle pour fil (21) du dispositif d'aide à l'insertion de fil-guide (10).

10. Dispositif d'aide à l'insertion de fil-guide (10) en association avec le réceptacle pour fil (2) et le fil-guide (1) selon la revendication 9, dans lequel le réceptacle pour fil (2) est fixé de façon amovible au support de réceptacle (21) du dispositif d'aide à l'insertion de fil-guide (10).

11. Dispositif d'aide à l'insertion de fil-guide (10) en association avec le réceptacle pour fil (2) et le fil-guide (1) selon la revendication 9, dans lequel le dispositif d'aide à l'insertion de fil-guide (10) comprend en outre une pièce de fixation (17) sur la partie formant corps principal (11), dans lequel la pièce de fixation (17) saisit le réceptacle pour fil (2) .
